# EUROPÄISCHE PATENTSCHRIFT

(11) **EP 1 203 764 B1**
(45) Veröffentlichungstag und Bekanntmachung des Hinweises auf die Patenterteilung: **12.05.2004**
(21) Anmeldenummer: 02003690.1
(22) Anmeldetag: 09.09.1997
(51) Int. Cl.: C07C 257/18, C07C 257/20, C07C 271/64, A61K 31/155, A61P 11/00

(54) **Benzamidinderivate mit LTB4- antagonistischer Wirkung**
Benzamidine derivatives with LTB4-antagonistic activity
Dérivés de benzamidine ayant une activité antagoniste de LTB4

(30) Priorität: 10.09.1996 DE 19636689
(43) Veröffentlichungstag der Anmeldung: 08.05.2002
(62) Teilanmeldung aus: 97942007.2
(73) Patentinhaber: Boehringer Ingelheim Pharma GmbH & Co.KG, 55218 Ingelheim am Rhein (DE)
(72) Erfinder: Schromm, Kurt, 55218 Ingelheim am Rhein (DE); Anderskewitz, Ralf, 88471 Laupheim (DE); Renth, Ernst-Otto, 24105 Kiel (DE); Birke, Franz, 55218 Ingelheim am Rhein (DE); Jennewein, Hans Michael, 65193 Wiesbaden (DE); Meade, Christopher, John, Montague, 88437 Maselheim (DE)

(56) Entgegenhaltungen:
- WO-A-93/16036
- DE-A- 4 424 713
- DE-A- 4 424 714
- DE-A- 19 546 452

## Beschreibung

Die vorliegende Erfindung betrifft neue Benzamidinderivate, Verfahren zu ihrer Herstellung sowie ihre Verwendung als Arzneimittel. Die neuen Benzamidinderivate entsprechen der Formel **1,** 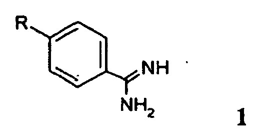 wobei R ausgewählt aus der Gruppe bestehend aus den folgenden Resten ist: 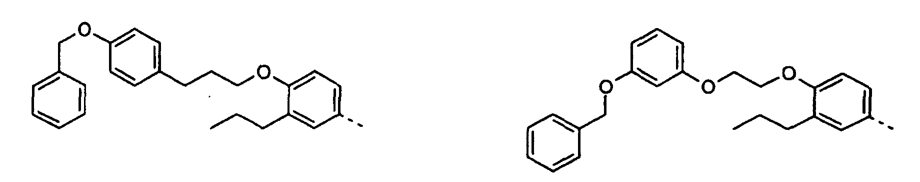 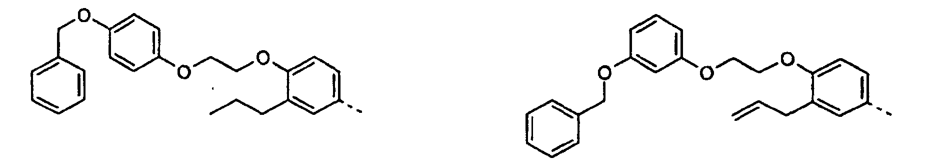 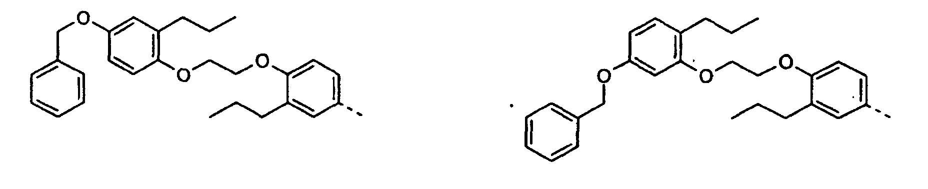 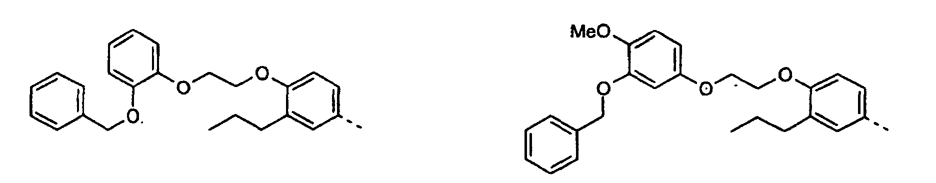 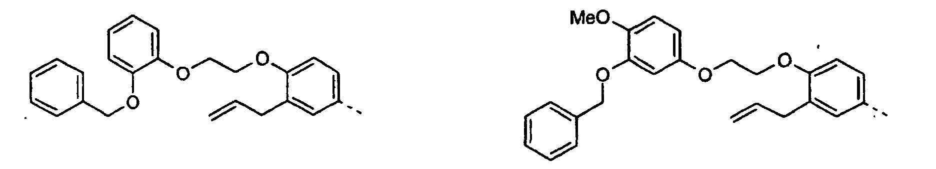 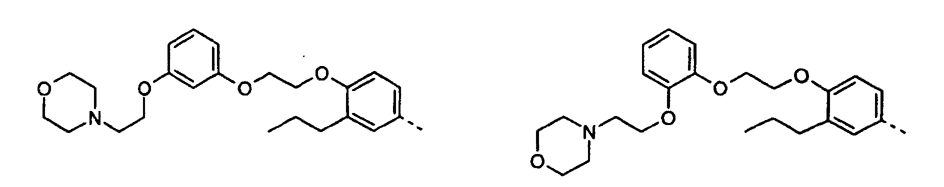 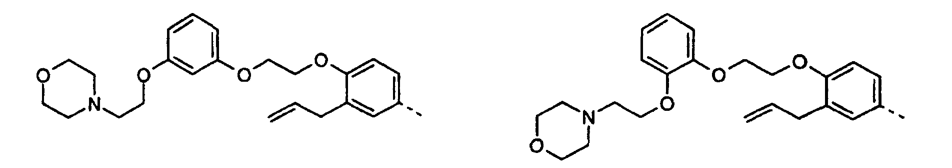 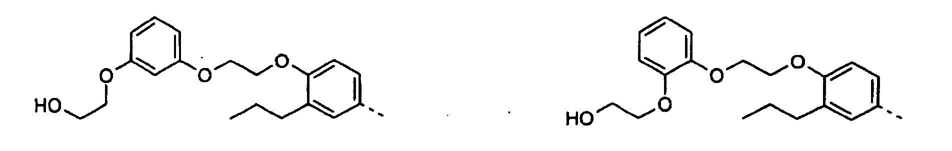 gegebenenfalls in Form der einzelnen optischen Isomeren, Mischungen der einzelnen Enantiomeren oder Racemate sowie in Form der freien Basen oder der entsprechenden Säureadditionssalze mit pharmakologisch unbedenklichen Säuren.

Wie gefunden wurde, zeichnen sich die Verbindungen der Formel **1** durch vielfältige Anwendungsmöglichkeiten auf therapeutischem Gebiet aus. Hervorzuheben sind solche Anwendungsmöglichkeiten, für welche die LTB4-rezeptorantagonistischen Eigenschaften eine Rolle spielen. Hier sind insbesondere zu nennen:

Arthritis, Asthma, chronische obstruktive Lungenerkrankungen, etwa chronische Bronchitis, Psoriasis, Colitis ulcerosa, durch nichtsteroidale Antiphlogistika induzierte Gastro- oder Enteropathie, cystische Fibrose, Alzheimer-Krankheit, Schock, Reperfusionsschäden/Ischämien, Atherosklerose, Multiple Sklerose.

Auch lassen sich mit den neuen Verbindungen Krankheiten oder Zustände behandeln, bei denen die Passage von Zellen aus dem Blut über das vaskuläre Endothelium in das Gewebe von Bedeutung ist (etwa Metastasis) oder Krankheiten und Zustände, bei denen die Kombination des LTB4 oder eines anderen Moleküls (beispielsweise 12-HETE) mit dem LTB4-Rezeptor einen Einfluss auf die Zell-Proliferation hat (etwa chronische myelozytische Leukämie).

Die neuen Verbindungen können auch in Kombination mit anderen Wirkstoffen angewendet werden, etwa solchen, die für dieselben Indikationen Verwendung finden, oder z.B. mit Antiallergika, Sekretolytika, β2-Adrenergika, inhalativ anwendbaren Steroiden, Antihistaminika und/oder PAF-Antagonisten. Die Verabreichung kann topisch, oral, transdermal, nasal, parenteral oder inhalativ erfolgen.

Zur pharmakolgischen und biochemischen Untersuchung der Wirkungsverhältnisse eigene sich Tests, wie sie beispielsweise in der WO 93/16036, S 15 bis 17 - auf die hier inhaltliche Bezug genommen wird - offenbart sind.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 10 und 500 mg, vorzugsweise zwischen 20 und 250 mg. Bei inhalativer Anwendung werden dem Patienten zwischen etwa 0,5 und 25, vorzugsweise zwischen etwa 2 und 20 mg Wirkstoff zugeführt.

Inhalationslösungen enthalten im Allgemeinen zwischen etwa 0,5 und 5 % Wirkstoff. Die neuen Verbindungen können in üblichen Zubereitungen verabreicht werden, etwa als Tabletten, Dragées, Kapseln, Oblaten, Pulver, Granulate, Lösungen, Emulsionen, Sirupe, Inhalationsaerosole, Salben, Suppositorien.

### FORMULIERUNGSBEISPIELE

Die nachstehenden Beispiele zeigen einige Möglichkeiten für die Formulierung der Darreichungsformen:
1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.
2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.
3. Inhalationspulver
Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 33 45 722, auf die hiermit inhaltlich Bezug genommen wird, inhaliert.

Die neuen Verbindungen können nach folgenden konventionellen Methoden hergestellt werden, die an sich aus dem Stand der Technik bekannt sind.

Reduktion eines der Formel **2**, 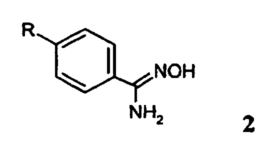 worin R die oben angegebene Bedeutung habt. Für die Reduktion des Amidoxims eignet sich die katalytische Hydrierung, insbesondere mit Raney-Nickel in einem niederen Alkohol, z.B. Methanol. Zweckmäßigerweise wird das Amidoxim der Formel **2** unter Zugabe der berechneten Menge derjenigen Säure, deren Salz als Endprodukt gewünscht wird, in Methanol gelöst und bei Raumtemperatur unter leichtem Druck, z.B. bei 5 bar, bis zur beendeten Wasserstoffaufnahme hydriert.

Umsetzung von Iminoestem der Formel **3** 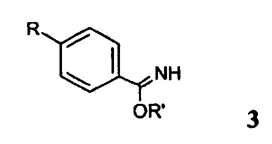 in der R die in Anspruch 1 angegebene Bedeutung hat und R' einem niederen Alkylrest entspricht; mit Ammoniak umsetzt. Die Umsetzung erfolgt zweckmäßig in einem organischen Lösungsmittel bei Temperaturen zwischen etwa 0°C und der Siedetemperatur des Reaktionsgemischs, vorzugsweise zwischen Raumtemperatur und etwa 100°C bzw. der Siedetemperatur, soweit diese niedriger ist. Geeignete Lösungsmittel sind polare Lösungsmittel wie Methanol, Ethanol, Propanole.

Die erfindungsgemäßen Verbindungen sind ausgehend von aus dem Stand der Technik bekannten Verbindungen u.a. nach den in den folgenden Referenzbeispielen beschriebenen Verfahren herstellbar. Verschiedenartige, andere Ausgestaltungen der Verfahren werden für den Fachmann aus der vorliegenden Beschreibung ersichtlich. Es wird jedoch ausdrücklich darauf hingewiesen, dass diese Beispiele und die diesen zugeordnete Beschreibung lediglich zum Zweck der Erläuterung vorgesehen und nicht als Einschränkung der Erfindung anzusehen sind.

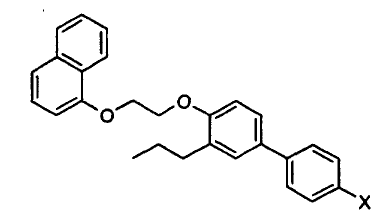

**Amidoxim: X entspricht C(=NOH)NH**_{**2**}
5,3 g des Nitrils der obigen Formel (X = CN) wird zusammen mit 98 ml Ethanol, 3,65 g Hydroxylamin x HCl, 2,9 g Na₂CO₃ und 21 ml Wasser 5 Stunden am Rückfluss gekocht. Danach wird bis zur Trockne abdestilliert und der Rückstand mit Wasser verrührt und abgesaugt. 5,7 g wird in 25 ml Acetonitril aufgeschlämmt und mit 0,85 ml Methansulfonsäure versetzt, erwärmt und wieder abgekühlt. Nach dem Absaugen erhält man 6,4 g des Methansulfonats als weiße Kristalle.

**Amidin: X entspricht C(=NH)=NH**_{**2**}
6,4 g des Amidoxims (X entspricht C(=NOH)-NH₂) als Methansulfonat wird in 100 ml Methanol gelöst und mit Raney-Nickel als Katalysator unter Normalbedingungen bis zur 100 %igen Wasserstoffaufnahme hydriert. Nach Absaugen wird eingeengt und der Rückstand aus Methanol umkristallisiert. Ausbeute: 3,8 g der Amidinverbindung als Methansulfonat. Fp. 228 - 30°C.

**Ethoxycarbonylamidin: X = C(NCOOC**_{**2**}**H**_{**5**}**)-NH**_{**2**}
2,6 g des Amidinmethansulfonats wird mit 1,6 ml Triethylamin in 50 ml Essigester suspendiert und dazu lässt man 0,6 ml Chlorameisensäureethylester in 5,5 ml Essigester bei Raumtemperatur in etwa 15 Minuten zutropfen. Das Reaktionsgemisch wird dann dreimal mit Wasser gewaschen, mit Na₂SO₄ getrocknet und eingedampft. Nach Umkristallisieren aus Acetonitril erhält man die Ethoxycarbonylamidinverbindung vom Fp. 142 - 144°C.

Gemäß dieser Referenzvorschrift werden u.a. folgende Verbindungen erhalten:

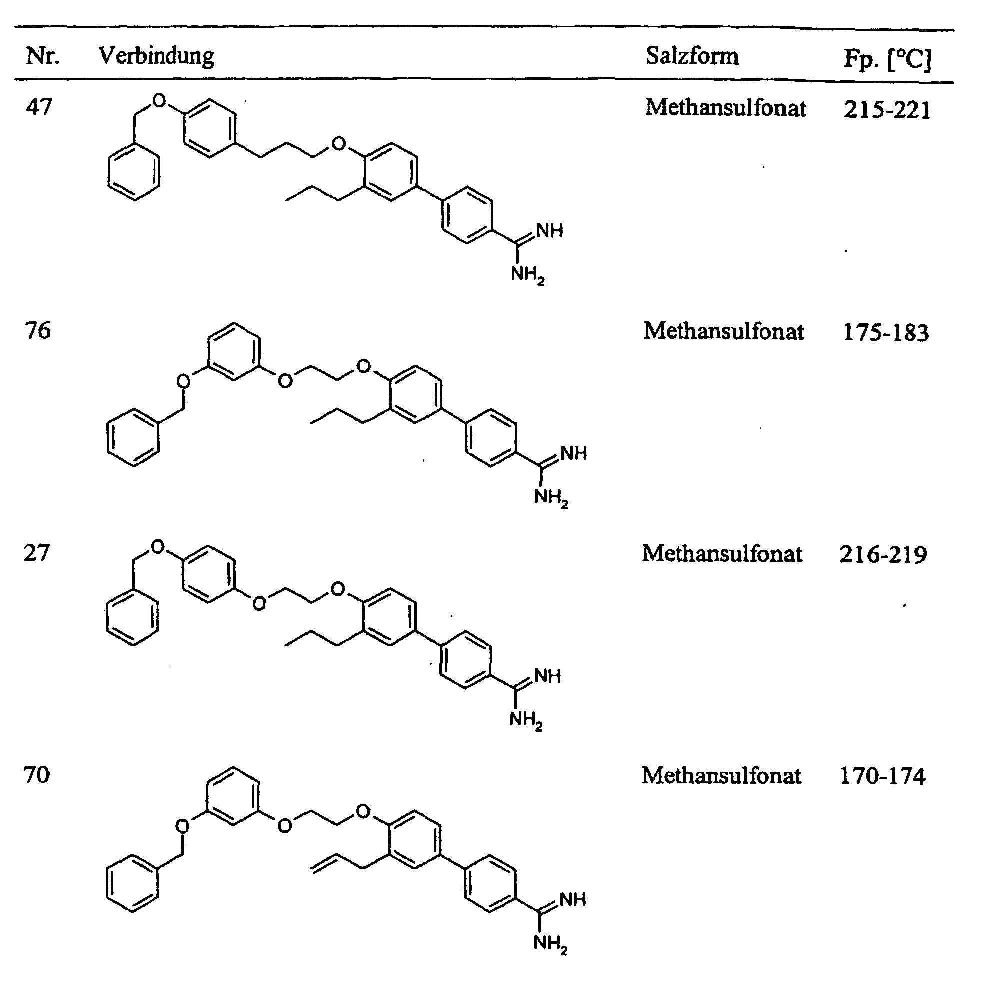

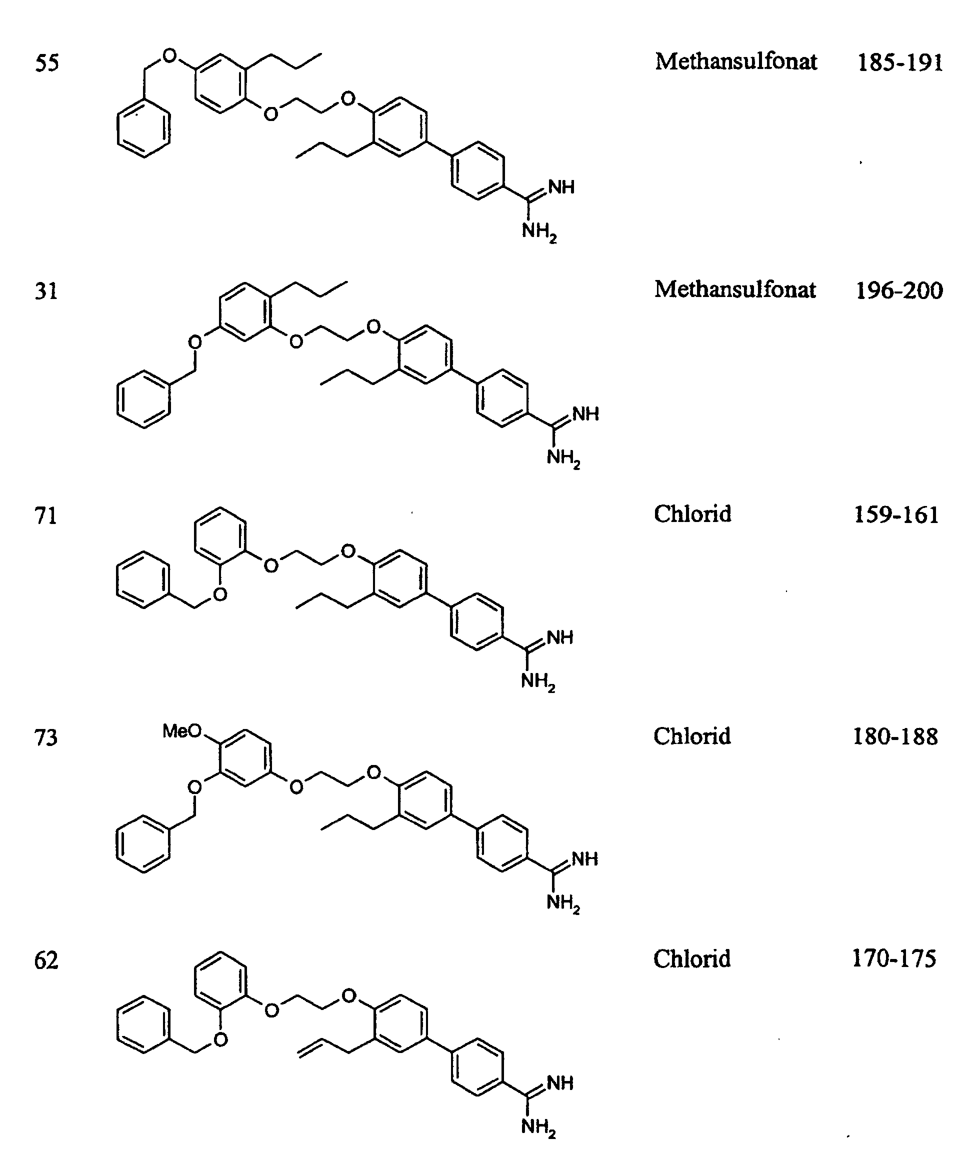

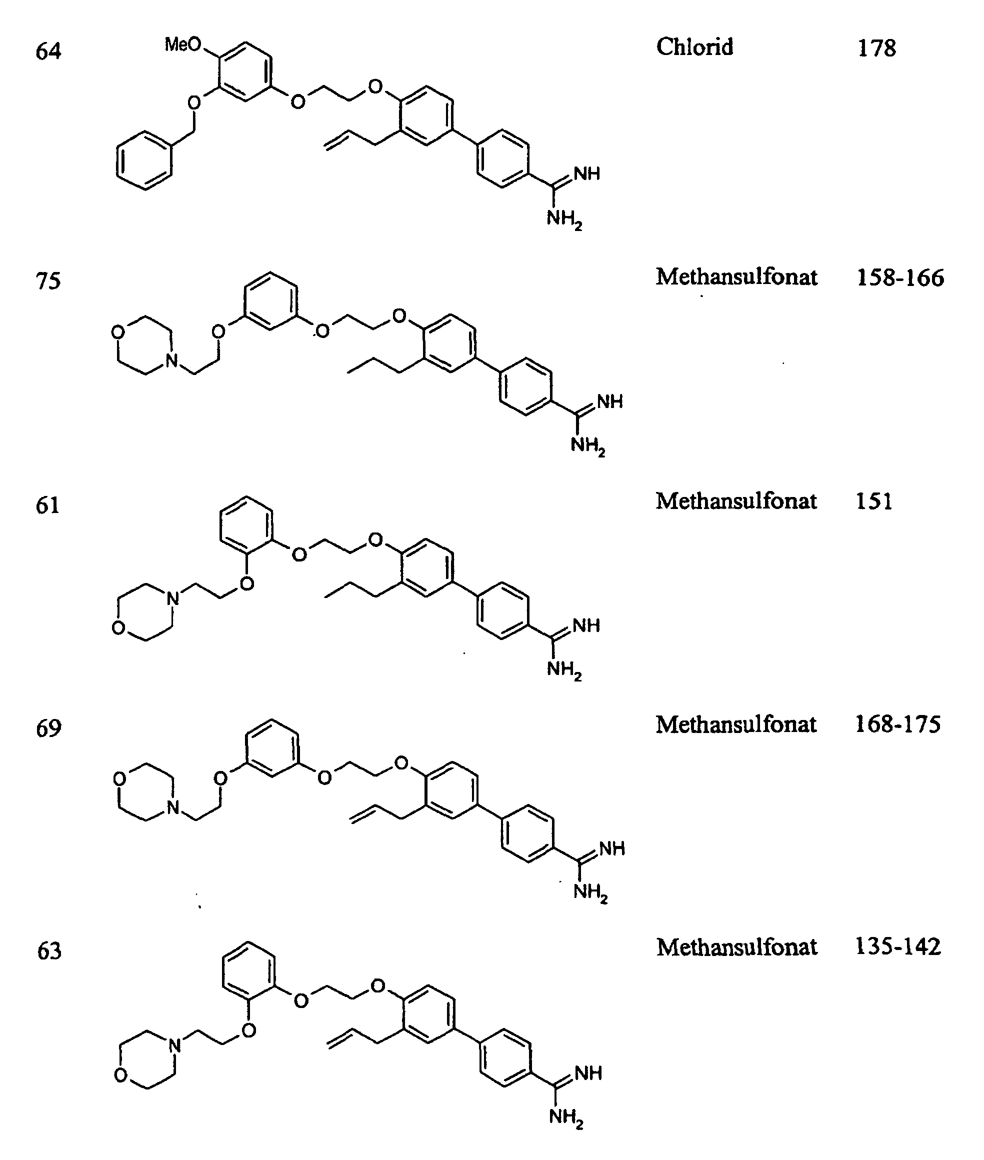

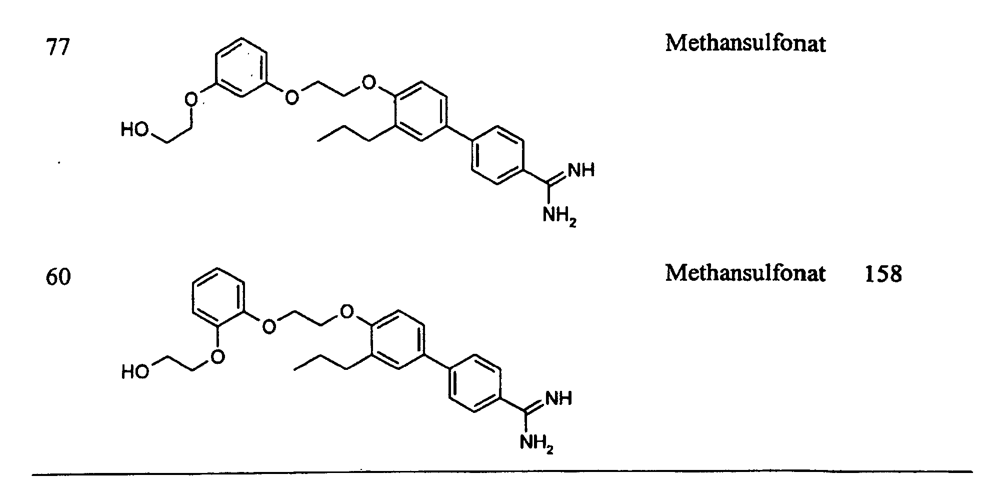

## Patentansprüche

1. Benzamidinderivate der Formel 1, 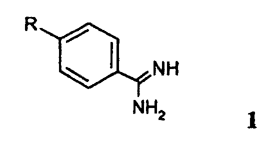 wobei R ausgewählt aus der Gruppe bestehend aus den folgenden Resten ist:

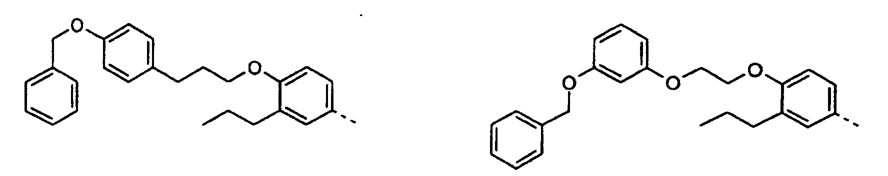

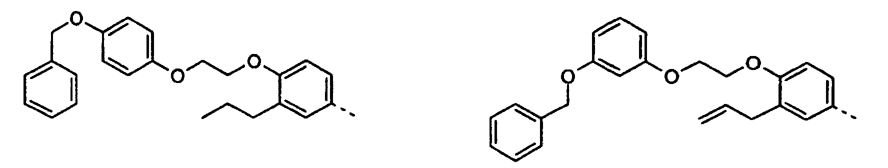

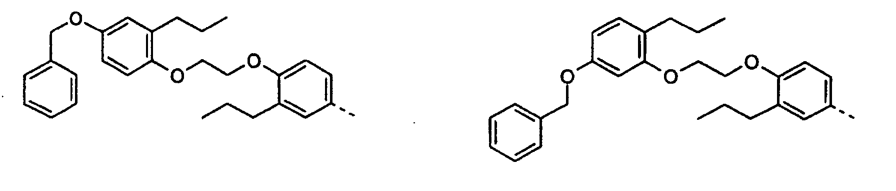

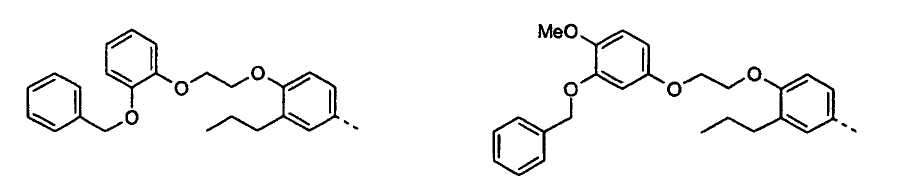

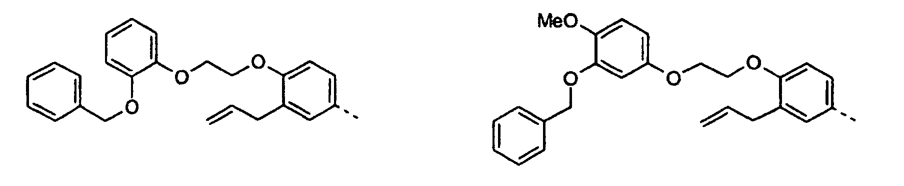

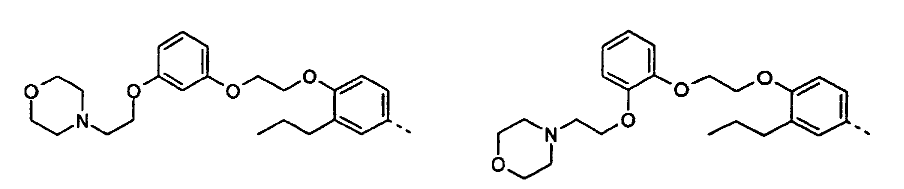

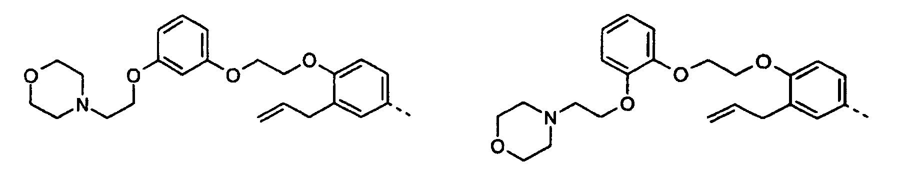

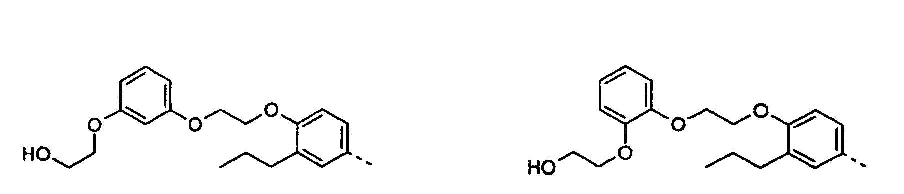

2. Verfahren zur Herstellung der Verbindungen nach Anspruch 1, **dadurch gekennzeichnet, dass** man Amidoxime der Formel **2**, 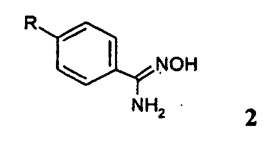 worin R die in Anspruch 1 angegebene Bedeutung hat, auf an sich bekannte Weise reduziert.

3. Verfahren zur Herstellung der Verbindungen nach Anspruch 1, **dadurch gekennzeichnet, dass** man Verbindungen der Formel **3**, 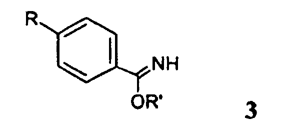 in der R die in Anspruch 1 angegebene Bedeutung hat und R' einem niederen Alkylrest entspricht; mit Ammoniak umsetzt.

4. Pharmazeutische Zubereitung, **gekennzeichnet durch** einen Gehalt an einer der Verbindungen nach Anspruch 1 und deren Säureadditionssalze neben üblichen Hilfs- und Trägerstoffen.

5. Verbindungen der Formel **1** nach Anspruch 1 zur Verwendung als Arzneimittel.

6. Verwendung von Verbindungen der Formel **1** nach der Anspruch 1 zur Herstellung eines Arzneimittels mit LTB₄-antagonistischer Wirkung.

7. Verwendung von Verbindungen der Formel **1** nach Anspruch 1, deren Stereoisomere sowie deren Säureadditionssalze zur Herstellung eines Medikaments zur therapeutischen Behandlung von Arthritis, Asthma, chronischer obstruktiver Lungenerkrankung wie chronischer Bronchitis, Psoriasis, Colitis ulcerosa, durch nichtsteroidale Antiphlogistika induzierter Gastro- oder Enteropathie, cystischer Fibrose, Alzheimer-Krankheit, Schock, Reperfusionsschäden/Ischämien, Atheriosklerose, multipler Sklerose.

## Claims

1. Benzamidine derivatives of formula 1 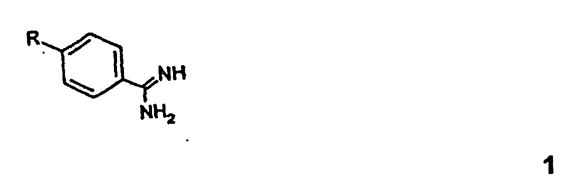 wherein R is selected from the group consisting of the following radicals:

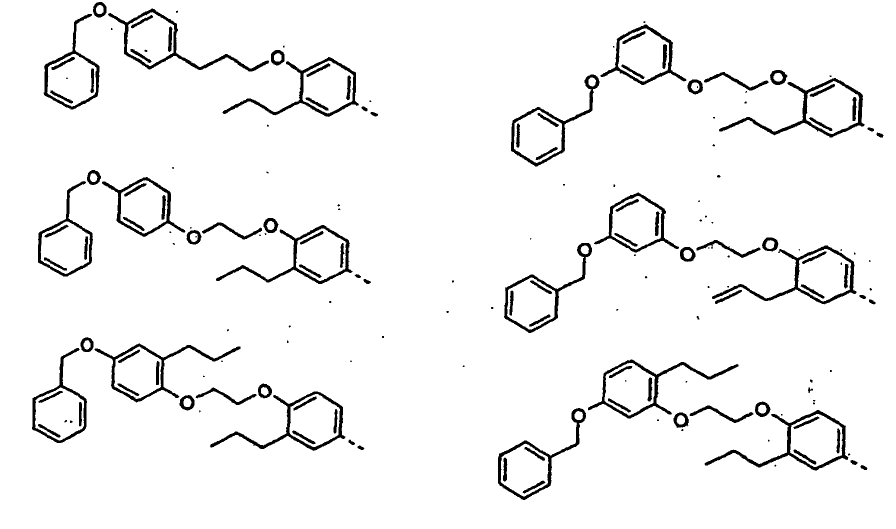

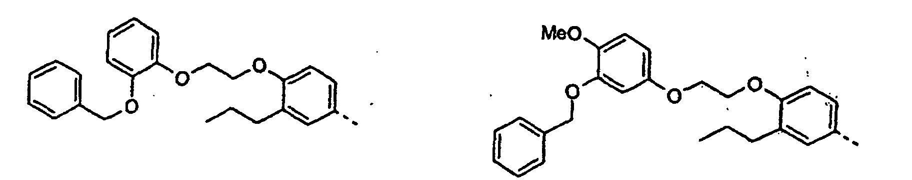

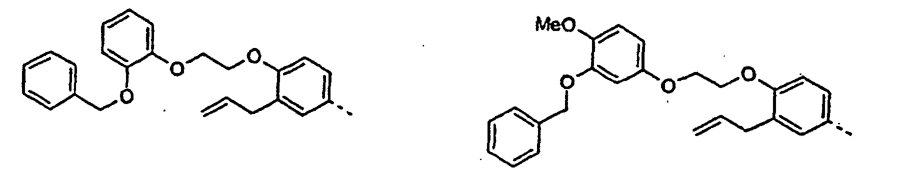

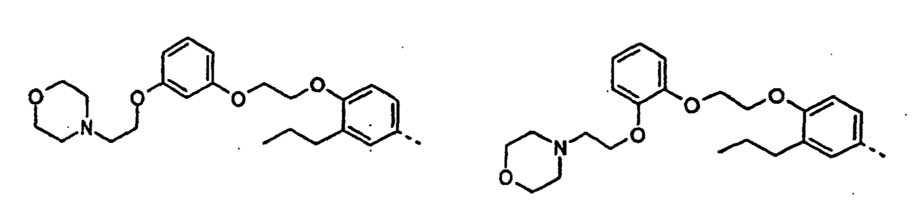

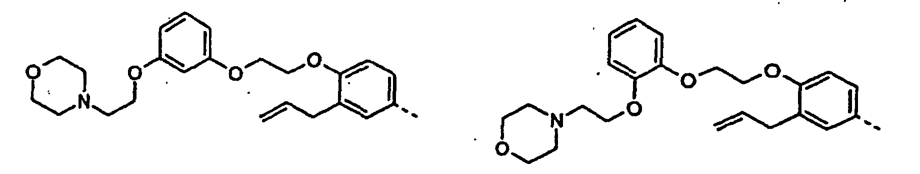

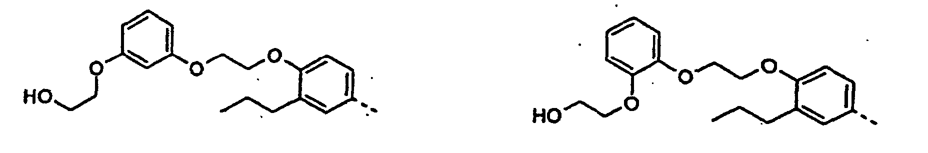

2. Process for preparing the compounds according to claim 1,
**characterised in that** amidoximes of formula **2** 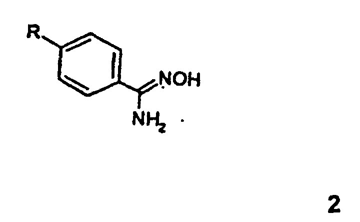 wherein R has the meaning given in claim 1, are reduced in a manner known *per se*.

3. Process for preparing the compounds according to claim 1,
**characterised in that** compounds of formula **3** 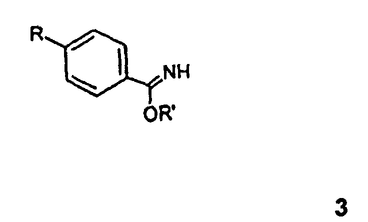 wherein R has the meaning given in claim 1 and R' corresponds to a lower alkyl group are reacted with ammonia.

4. Pharmaceutical preparation, **characterised in that** it contains a compound according to claim 1 and the acid addition salts thereof together with conventional excipients and carriers.

5. Compounds of formula **1** according to claim 1 for use as pharmaceutical compositions.

6. Use of compounds of formula **1** according to claim 1 for preparing a pharmaceutical composition with an LTB₄-antagonistic activity.

7. Use of compounds of formula **1** according to claim 1, the stereoisomers and the acid addition salts thereof, for the preparation of a medicament for the therapeutic treatment of arthritis, asthma, chronic obstructive pulmonary disease such as chronic bronchitis, psoriasis, ulcerative colitis, gastropathy or enteropathy induced by nonsteroidal anti-inflammatories, cystic fibrosis, Alzheimer's disease, shock, reperfusion damage/ischaemia, atherosclerosis and multiple sclerosis.

## Revendications

1. Dérivés de benzamidine de formule 1 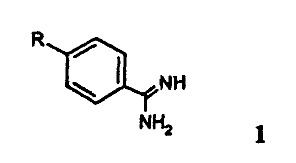 où R est choisi dans le groupe consistant en les restes suivants :

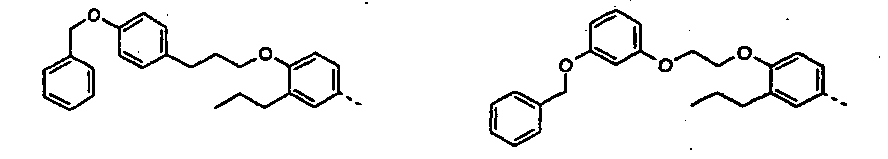

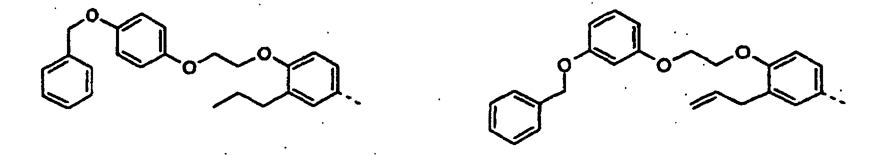

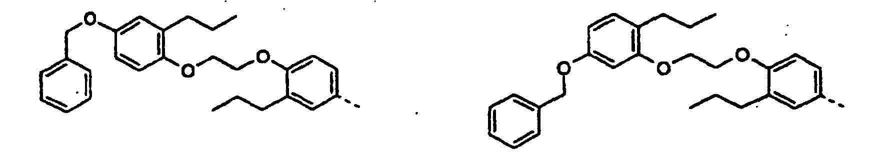

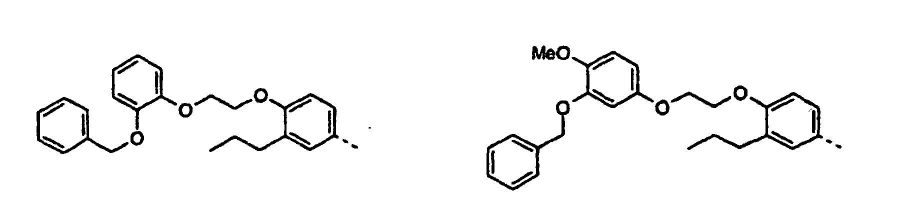

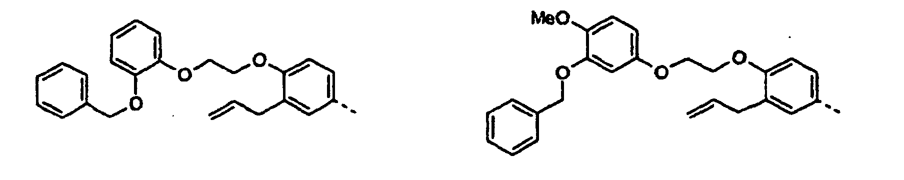

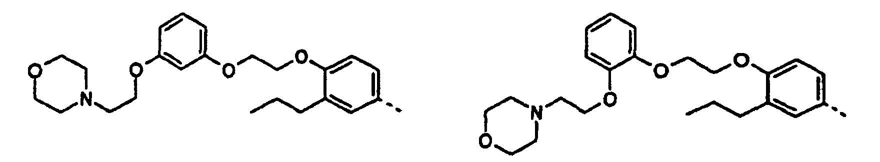

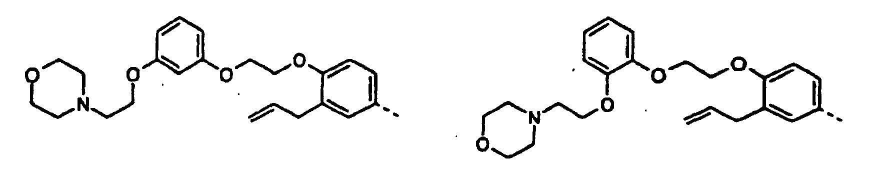

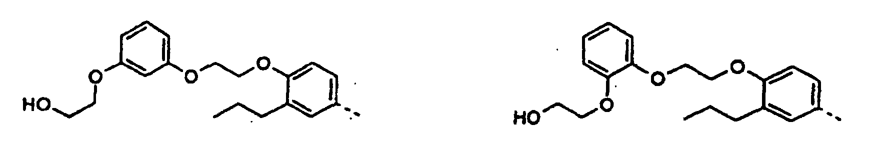

2. Procédé de préparation des composés selon la revendication 1 **caractérisé en ce que** l'on réduit de manière connue en soi des amidoximes de formule 2 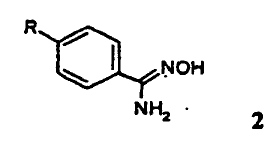 où R a la signification indiquée dans la revendication 1.

3. Procédé de préparation des composés selon la revendication 1 **caractérisé en ce que** l'on fait réagir avec l'ammoniac des composés de formule 3 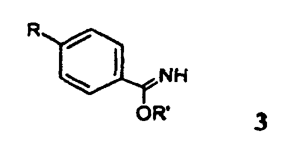 où R a la signification indiquée dans la revendication 1 et R' correspond à un reste alkyle inférieur.

4. Préparation pharmaceutique **caractérisée par** une teneur en l'un des composés selon la revendication 1 et ses sels d'addition d'acide, outre des adjuvants et supports habituels.

5. Composés de formule 1 selon la revendication 1 destinés à être utilisés comme médicament.

6. Utilisation de composés de formule 1 selon la revendication 1 pour la production d'un médicament à effet antagoniste de LTB₄.

7. Utilisation de composés de formule 1 selon la revendication 1, de leurs stéréoisomères ainsi que de leurs sels d'addition d'acide pour la production d'un médicament pour le traitement thérapeutique de l'arthrite, de l'asthme, des maladies pulmonaires obstructives chroniques comme la bronchite chronique, du psoriasis, de la colite ulcéreuse, des gastro- ou entéropathies dues à des antiphlogistiques non stéroïdiens, de la fibrose kystique, de la maladie d'Alzheimer, du choc, des lésions de reperfusion/ischémies, de l'athérosclérose, de la sclérose en plaques.
